# EUROPÄISCHE PATENTSCHRIFT

(11) **EP 0 636 037 B1**
(45) Veröffentlichungstag und Bekanntmachung des Hinweises auf die Patenterteilung: **05.02.1997**
(21) Anmeldenummer: 93908804.3
(22) Anmeldetag: 16.04.1993
(51) Int. Cl.: A61M 16/01

(54) **ANÄSTHESIEMASCHINE**
ANAESTHETIST'S EQUIPMENT
APPAREIL D'ANESTHESIE

(30) Priorität: 16.04.1992 DE 4212904
(43) Veröffentlichungstag der Anmeldung: 01.02.1995
(73) Patentinhaber: Obermayer, Anton, Dr.-Ing., 91054 Erlangen (DE)
(72) Erfinder: Obermayer, Anton, Dr.-Ing., 91054 Erlangen (DE)
(74) Vertreter: Patentanwälte Eisele, Otten & Roth
(86) Internationale Anmeldenummer: DE9300348
(87) Internationale Veröffentlichungsnummer: WO9320875

(56) Entgegenhaltungen:
- WO-A-82/00766
- DE-A- 2 945 485
- DE-A- 3 820 165
- DE-A- 3 900 276
- US-A- 4 231 362

## Beschreibung

Die Erfindung betrifft eine Anästhesiemaschine nach dem Oberbegriff des Anspruchs 1.

### Stand der Technik:

In der Medizintechnik unterscheidet man zwischen Anästhesiemaschinen und Beatmungssystemen.

Reine Beatmungssysteme sind beispielsweise in Form von Atemhilfegeräten bekannt geworden, wie sie beispielsweise in der DE 38 20 043 A1, der DE 37 12 389 A1 oder der EP 0 112 979 B1 beschrieben sind. Beatmungsgeräte oder Atemhilfegeräte dienen zur Beatmung von Patienten vor oder auch nach einer Operation in den Fällen, wo die eigene Atemfähigkeit des Patienten nicht ausreicht.

Demgegenüber dient eine Anästhesiemaschine der speziellen Behandlung des Patienten während einer Operation. Die Narkose wird bei größeren chirurgischen Eingriffen in der Regel als sogenannte Inhalationsnarkose durchgeführt. Dabei wird dem Patienten über eine Anästhesiemaschine das Narkosegasgemisch zugeführt, welches aus Sauerstoff, Lachgas und einem dampfförmigen Anästhetik besteht. Gleichzeitig wird eine Beatmung durchgeführt. Ein Narkose-Beatmungsgerät ist beispielsweise in der EP 0 058 706 B1 beschrieben.

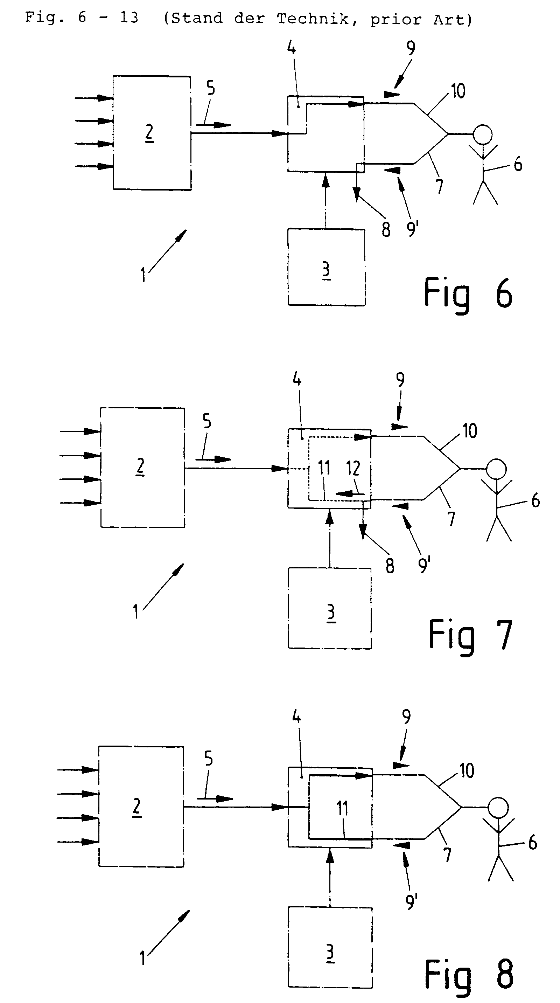

In den Figuren 6 bis 13 wird der für die vorliegende Erfindung relevante Stand der Technik zum besseren Verständnis im nachfolgenden näher erläutert.

### Beschreibung von bekannten Anästhesiemaschinen in verschiedenen Arbeitstechniken:

Wie in den Figuren 6 bis 8 dargestellt, besteht eine bekannte Anästhesiemaschine 1 im wesentlichen aus drei Hauptgruppen, nämlich einem Narkosegerät 2, einem Beatmungsteil 3 und einem Narkosesystem 4. Das Narkosegerät 2 mit Hochdruckgasversorgung dient der Herstellung eines Frischgasflusses 5, d. h. einem Narkosegasgemisch, bestehend aus Sauerstoff, Lachgas sowie dem Narkosemittel. Das Frischgas 5 wird kontinuierlich oder diskontinuierlich in das Narkosesystem 4 geleitet.

Das Narkosesystem 4 stellt die zentrale Funktionseinheit einer Anästhesiemaschine dar, mit der der eigentliche Gasaustausch zwischen der Maschine und dem Patienten bewerkstelligt wird. Abhängig vom Grad der Betäubung, d. h. der Narkosetiefe, wird der Gasaustausch durch den Patienten in Form der Spontanatmung, oder bei Ausfall der Spontanatmung durch eine maschinelle oder manuelle Beatmung erreicht. Dabei erfolgt die Beatmung durch den zusätzlichen Beatmungsteil 3.

Bei den heute gebräuchlichen Narkosesystemen 4 lassen sich abhängig von der Wiederverwendung des in das Narkosesystem zurückgeführten Ausatemgas des Patienten 6 drei verschiedene Grundtypen unterscheiden, die in den Figuren 6 bis 8 dargestellt sind.

Bei dem in der Figur 6 dargestellten "halboffenen Narkosesystem" wird das ausgeatmete, vom Patienten 6 in das Narkosesystem 4 über die Leitung 7 zurückströmende Gas nicht wieder verwendet, sondern komplett über die Narkosegasabsaugung 8 aus dem System entfernt. Die vom Patienten benötigte Gasmenge (Atemgas 9) muß dem Narkosesystem 4 als Frischgas 5 vom Narkosegerät 2 über die Zuführleitung 10 zugeführt werden. Die Gaszuführleitung 10 wird als inspiratorischer, die Gasabfuhrleitung 7 als exspiratorischer Zweig bezeichnet.

Bei dem in Figur 7 dargestellten sogenannten "halbgeschlossenen Narkosesystem" wird ein Teil des ausgeatmeten Gases wieder verwendet, d. h. die dem Patienten inspiratorisch zugeführte Gasmenge (= Atemhubvolumen) besteht zum Teil aus vorher ausgeatmeten Exspirationsgas und nur zum Teil aus Frischgas. In Figur 7 ist hierfür ein gestrichelter Kreislauf 11 eingezeichnet, d. h. das Exspirationsgas aus der Exspirationsleitung 7 geht zum Teil in die Narkosegasabsaugung 8 und zum anderen Teil (Pfeil 12) zurück zum inspiratorischen Zweig 10. Durch diese Verwendung des exspiratorischen Gases läßt sich eine erhebliche Frischgaseinsparung und gleichzeitig eine Klimatisierung des inspiratorischen Atemgases erzielen. Die Klimatisierung des Atemgases 9 erfolgt dabei durch Mischung des Frischgases 5 mit dem exspiratorischen Gas, welches 100 % Luftfeuchtigkeit und eine Temperatur von 37 Grad aufweist. Durch die Klimatisierung erfolgt demnach eine Erwärmung sowie eine Befeuchtung des inspiratorischen Atemgases. Der geringere Bedarf an Frischgas 5 macht sich auch kostenmäßig bemerkbar.

Bei dem in Figur 8 dargestellten sogenannten "geschlossenen Narkosesystem" wird das exspirierte Atemgas 9' in der Exspirationsleitung 7 im Narkosesystem 4 vollständig gesammelt und dem Patienten 6 in der nächsten Inspiration wieder zugeführt. Dies wird durch den geschlossenen Kreislauf 11 in Figur 8 dargestellt, d. h. eine Narkosegasabsaugung fehlt vollständig. Der erforderliche Frischgasfluß 5 wird bei diesem System gemaß Figur 8 minimal, da nur der vom Patienten verbrauchte Sauerstoff und das gespeicherte Lachgas und Narkosemittel zugeführt werden muß. Der Frischgasfluß 5 wird demnach minimal, was auch eine geringe Umweltbelastung durch die Narkosegase durch eine fehlende Narkosegasabsaugung bewirkt. Gleichzeitig wird eine optimale Klimatisierung, d. h. Erwärmung und Befeuchtung des inspiratorischen Atemgases 9 erzielt.

Bei allen Narkosesystemen gemäß Figur 6 bis 8 ist zum Beispiel bei einer Operation eine künstliche Beatmung durch das Beatmungsgerät 3 erforderlich, da aufgrund der Narkose das eigene Atemsystem ausgeschaltet ist. Dabei wird in der inspiratorischen Phase das Atemgas mit einem geringen Überdruck in der Größenordnung von 10 bis 20 mbar dem Patienten zugeführt. In der exspiratorischen Phase wird ein Exspirationsventil geöffnet und eine Druckentlastung des Atemdrucks bis auf Umgebungsdruck durchgeführt (Peep = 0). In Sonderfällen kann auch ein künstlicher Überdruck in der Exspirationsphase gewählt werden, d. h. das Exspirationsventil ist auf einen Peep-Druck p > 0, z. B. p (Peep) ≈ 2 bis 5 mbar eingestellt.

Die am Markt befindlichen Narkosemaschinen sind in der Regel entweder nur für das halboffene Narkosesystem oder nur für das halbgeschlossene Narkosesystem oder umschaltbar für beide Systeme konzipiert.

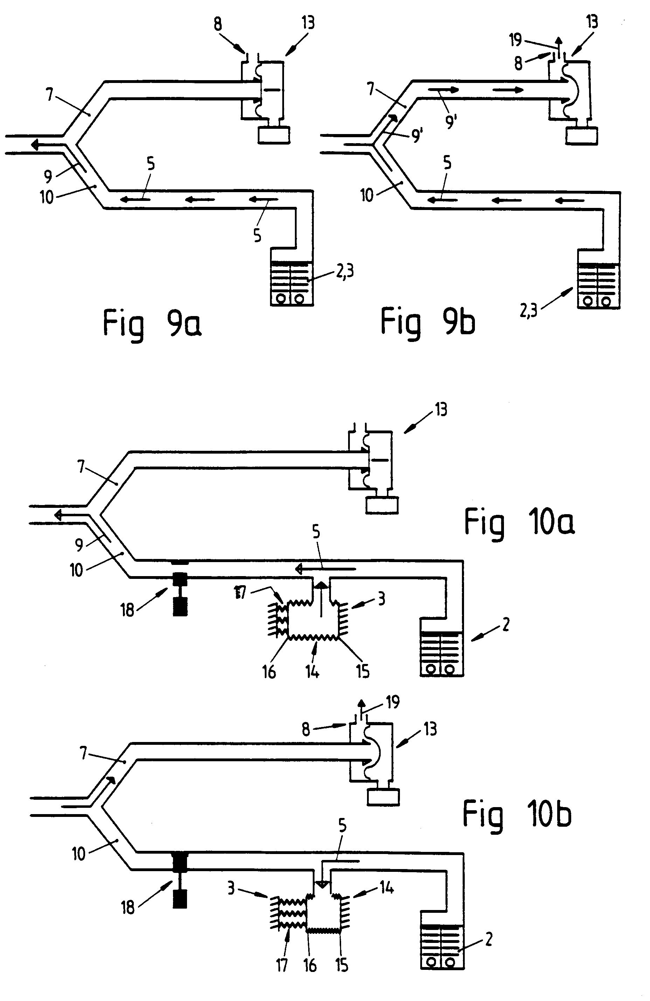

Für den Beatmungsteil werden für das sogenannte "halboffene Narkosesystem" entweder sogenannte "Flowzerhacker" oder sogenannte "Respiratoren mit Druckgasspeicher" verwendet. In den Figuren 9a, 9b ist ein solcher "Flowzerhacker" mit angesteuertem Exspirationsventil dargestellt. Dabei zeigt Figur 9a die inspiratorische Phase, die Figur 9b die exspiratorische Phase. Die in den Figuren 6 bis 8 verwendeten Bezugszeichen werden für gleiche Teile auch im folgenden weiterverwendet.

In der inspiratorischen Phase gemäß Figur 9a wird bei geschlossenem Exspirationsventil 13 die Patientenlunge durch das vom kombinierten Narkosegerät 2 mit Beatmungsmaschine 3 und Hochdruckgasversorgung kommende Frischgas 5 über den inspiratorischen Zweig 10 befüllt. In der exspiratorischen Phase gemäß Figur 9b kann der Patient bei geöffnetem Exspirationsventil 13 über die Exspirationsleitung 7 ausatmen. Dabei wird in der Exspirationsphase der anhaltende Frischgasfluß 5 ebenfalls über das Exspirationsventil 13 ins Freie oder in eine Narkoseabsaugung 8 abgeleitet (Pfeil 19). Eine Rückführung des exspirierten Atemgases ist nicht vorgesehen. Dieses System ist deshalb unwirtschaftlich und teuer und bedarf einer zusätzlichen Einrichtung für die Klimatisierung des Inspirationsgases.

Auch bei dem bekannten halboffenen Narkosesystem mit einem Respirator mit Druckgasspeicher gemäß der Darstellung nach Figur 10a, 10b erfolgt keine Wiederverwendung des exspirierten Atemweggases des Patienten. Wie in Figur 10a für die inspiratorische Phase dargestellt, weist dieses Narkosesystem neben dem Narkosegerät 2 einen zusätzlichen Druckgasspeicher 14 als Beatmungsteil 3 auf, der aus einer festen Platte 15 und einer beweglichen Platte 16 sowie einem Druckfedersystem 17 besteht. Weiterhin weist dieses halboffene Narkosesystem ein ansteuerbares Inspirationsventil 18 auf. In der inspiratorischen Phase gemäß Figur 10a ist das Inspirationsventil 18 geöffnet und das angesteuerte Exspirationsventil 13 geschlossen. Aufgrund des Druckgefälles zwischen dem Druckgasspeicher 14 und der Lunge des Patienten strömt das Frischgas 5 bei geöffnetem Inspirationsventil 18 über den inspiratorischen Zweig 10 zum Patienten. Die Befüllung des Beatmungsgerät bildenden Druckgasspeichers 14 erfolgt kontinuierlich oder diskontinuierlich durch die Hochdruckgasversorgung des Narkosegerätes 2 bis zu einem Druck von ca. 5 bar. Mit Hilfe des regelbaren Inspirationsventiles 18 ist eine sehr genaue Gasdosierung möglich, die die Beatmung auch von Neonaten gestattet. Neben der guten Regelbarkeit zeichnet sich dieses System durch hohe Gasdichtigkeit aus. Nachteilig ist jedoch der hohe Gasverbrauch und die fehlende Klimatisierung durch das halboffene Narkosesystem, denn das exspirierte Atemgas geht in der in Figur 10b dargestellten exspiratorischen Phase bei geöffnetem Exspirationsventil 13 vollständig an die Umgebung bzw. in der Narkoseabsaugung 8 verloren (Pfeil 19). Während der exspiratorischen Phase bleibt das Inspirationsventil 18 geschlossen und das Frischgas 5 strömt in den Druckgasspeicher 14 und lädt diesen wieder gegen die Kraft des Druckfedersystems 17 auf.

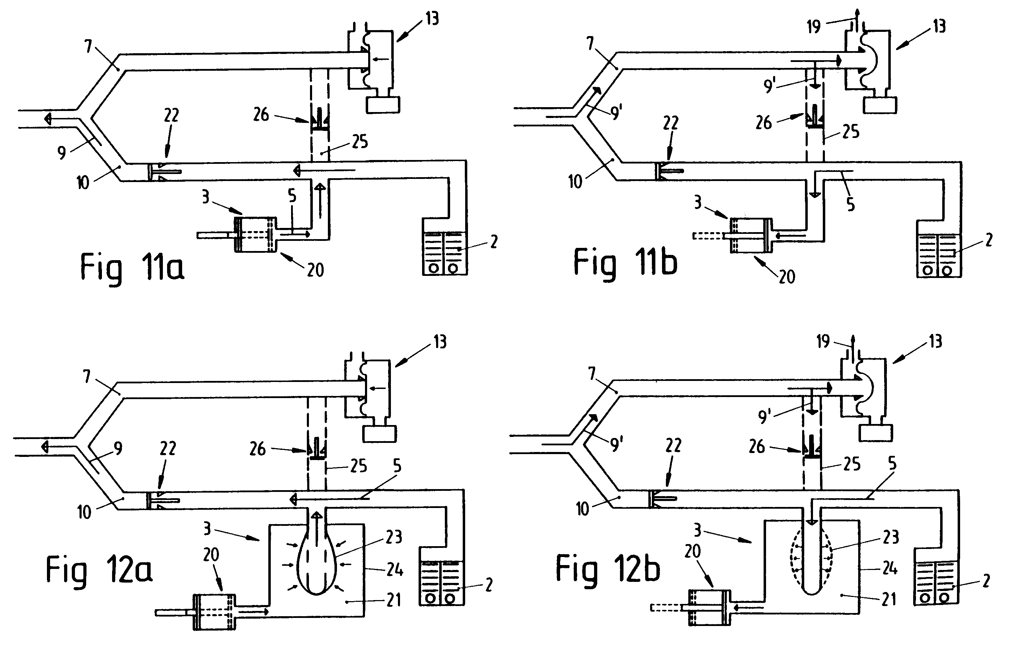

Eine weitere Lösung für ein "halboffenes Narkosesystem" nach dem bekannten System ist in den Figuren 11 und 12 angegeben, wobei die Figuren 11a, 12a jeweils die inspiratorischen und die Figuren 11b, 12b die exspiratorischen Phasen darstellen.

In der Figur 11 wird anstelle des Druckgasspeichers 14 in Figur 10 eine Kolbenpumpe 20 als Beatmungsteil 3 verwendet. Diese Kolbenpumpe 20 wird in Figur 12 durch das sogenannte "Bag in the bottle"-Prinzip erweitert, dessen Anordnung mit Bezugszeichen 21 gekennzeichnet ist. Anstelle des ansteuerbaren Inspirationsventils 18 in Figur 10 ist beim Ausführungsbeispiel nach den Figuren 11, 12 ein normales Rückschlagventil 22 vorgesehen.

Beim Ausführungsbeispiel nach Figur 11a wird in der inspiratorischen Phase die Kolbenpumpe 20 angetrieben und drückt das im zugehörigen Zylinderraum vorhandene Frischgas 5 über das sich selbständig öffnende Rückschlagventil 22 im Inspirationszweig 10 zum Patienten. In dieser Phase ist das ansteuerbare Exspirationsventil 13 geschlossen. Während der Exspirationsphase nach Figur 11b strömt das vom Patienten ausgeatmete Exspirationsgas 9' in den Exspirationszweig 7 und gelangt über das geöffnete Exspirationsventil 13 in die Umgebung (Pfeil 19). Während dieser Phase ist das Inspirationsventil 22 im Inspirationszweig 10 geschlossen. Aufgrund des lediglich als Rückschlagventil ausgebildeten Inspirationsventils 22 kann der erforderliche Beatmungsdruck grundsätzlich erst während des eigentlichen Inspirationsvorganges aufgebaut werden, dessen Höhe von der Lungenelastizität des Patienten und dem Strömungswiderstand abhängig ist. Durch Zurückziehen des Kolbens innerhalb der Kolbenpumpe 20 kann allerdings auch schon während der Exspirationsphase gemäß Figur 11b ein Teil des Frischgases 5 vom Narkosegerät 2 mit Hochdruckgasversorgung in die Kolbenpumpe 20 gefördert werden. Der sich aufbauende Druck in diesem inspiratorischen Zweig muß jedoch kleiner sein als der exspiratorische Druck im Exspirationszweig 7, damit das Rückschlagventil 22 geschlossen bleibt.

Das in Figur 12a, 12b gezeigte halboffene Narkosesystem arbeitet prinzipiell gleich wie zu Figur 11a, 11b beschrieben. Dabei wird das Frischgas 5 jeweils aus dem Balg oder Beutel 23 der Einrichtung 21 entnommen bzw. eingefüllt. Das aus der Kolbenpumpe 20 strömende Gas wird dabei innerhalb des Behälters 24 als "Primärsystem", das aus dem Beutel 23 strömende Gas als "Sekundärsystem" bezeichnet.

Das bekannte System nach Figur 11, 12 mit Kolbenpumpe 20 hat jedoch in der Praxis erhebliche Nachteile. Je nach Patiententyp (Kleinkind oder Baby mit einem Körpergewicht unter 2 kg oder Erwachsener) werden Inspirationsgasmengen zwischen 5 ml und 2500 ml benötigt. Dabei ist der Kolbenvorschub der Kolbenpumpe 20 nicht proportional der damit geförderten Gasmenge die beim Patienten ankommt, da zum einen das Gas komprimiert wird und zum anderen eine weitere zusätzliche Frischgaszufuhr aus dem Narkosegerät 2 erfolgt. Eine "Aufladung" des Systems ist aufgrund des Rückschlagventils 22 nicht möglich, so daß der inspiratorische Zweig quasi keiner Regelung unterliegt. Weiterhin ist aufgrund des halboffenen Narkosesystems gemäß Figur 11, 12 ebenfalls ein hoher Frischgasverbrauch vorhanden, verbunden mit einer fehlenden Klimatisierung ähnlich wie bei der Ausführung nach Figur 9 und 10.

Die bekannten Systeme nach Figur 11 und 12 können auch als sogenannte "halbgeschlossene Narkosesysteme" ausgebildet sein. In diesem Fall ist die in den Figuren 11 und 12 in gestrichelter Form eingezeichnete Verbindungsleitung 25 zwischen dem unteren inspiratorischen Zweig 10 und dem oberen exspiratorischen Zweig 7 vorgesehen. Ein zusätzliches Rückschlagventil 26 in der Verbindungsleitung 25 ist während der inspiratorischen Phase gemäß Figur 11a, 12a geschlossen und während der exspiratorischen Phase 11b, 12b geöffnet. Das exspiratorische Atemgas 9' kann demzufolge während der Exspirationsphase aus dem Exspirationszweig 7 über die Leitung 25 in den inspiratorischen Zweig 10 gelangen. Dies ist in Figur 11b, 12b mit den entsprechenden Pfeilen angedeutet. Auch bei einem solchen halbgeschlossenen Narkosesystem bleiben die zuvor genannten Nachteile mit der Einschränkung des zu hohen Frischgasverbrauches bestehen.

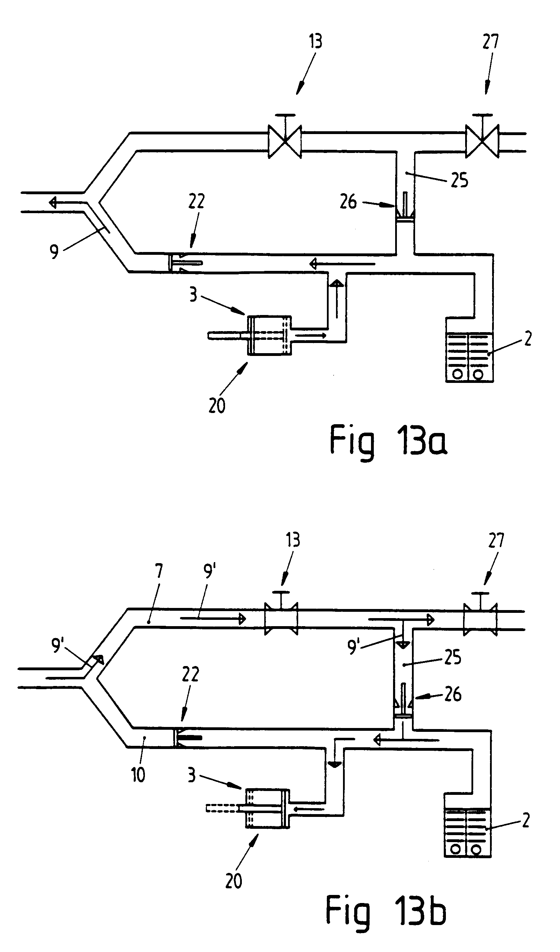

In den Figuren 13a, 13b ist schließlich ein bekanntes "halbgeschlossenes Narkosesystem" dargestellt, wie es aus dem Beispiel nach Figur 11a, 11b weiterentwickelt wurde. Dabei zeigt Figur 13a die inspiratorische und Figur 13b die exspiratorische Phase des Narkosesystems. Als Unterschied zum Ausführungsbeispiel nach Figur 11 ist im Ausführungsbeispiel nach Figur 13 im exspiratorischen Zweig ein ansteuerbares Exspirationsventil 13 und zusätzlich ein ansteuerbares Auslaßventil 27 vorgesehen. Durch diese Ventilanordnung kann mittels des ansteuerbaren Auslaßventils 27 das Maß des wieder zu verwendeten Exspirationsgases bestimmt werden. Wird das Auslaßventil 27 ganz geschlossen, wird das Narkosesystem zu einem "geschlossenen Narkosesystem" gemäß Figur 8 umfunktioniert, d. h. das komplette Exspirationsgas 9' gelangt in die Verbindungsleitung 25 zum inspiratorischen Zweig 10. Je nach Öffnungsgrad des Auslaßventils 27 wird das Maß der Umleitung des Exspirationsgases bestimmt. In Zwischenstellungen handelt es sich demnach um ein sogenanntes halbgeschlossenes Narkosesystem gemaß der prinzipellen Darstellung nach Figur 7. Die Anordnung nach Figur 13a, 13b hat demnach Regelungsvorteile gegenüber der Anordnung nach Figur 11a, 11b. Die prinzipelle Funktionsweise ist jedoch die gleiche.

Die vorstehende Beschreibung des Standes der Technik zeigt die prinzipelle Anwendung von Narkosesystemen in den jeweiligen halbgeoffenen, halbgeschlossenen oder geschlossenen Arbeitsweisen auf. Generell hat auch diese Vielzahl der bekannten Narkosesysteme allgemein den Nachteil, daß eine präzise Regelung des inspiratorischen Gases nicht möglich ist, da selbst bei einem ansteuerbaren Inspirationsventil 18 gemäß der bekannten Lösung nach Figur 10 eine genau Dosierung und Klimatisierung des inspiratorischen Gases nicht möglich ist. Durch den Druckgasspeicher 14 kann zwar das Frischgas während der exspiratorischen Phase sozusagen "vorgespannt" werden. Eine Zirkulation des exspiratorischen Atemgases ist bei diesem bekannten System jedoch nicht vorgesehen.

### Aufgabe der Erfindung:

Der Erfindung liegt die Aufgabe zugrunde, die bekannten Systeme dahingehend zu verbessern, daß eine Anästhesiemaschine für alle in den Figuren 6 bis 8 dargestellten Betriebsarten geschaffen wird, bei welcher möglichst wenig Frischgas benötigt und eine optimale Klimatisierung des inspiratorischen Gases erzielt wird.

Diese Aufgabe wird ausgehend von einer Anästhesiemaschine der einleitend bezeichneten Art durch die kennzeichnenden Merkmale des Anspruchs 1 gelöst.

In den Unteransprüchen sind vorteilhafte und zweckmäßige Weiterbildungen der Anästhesiemaschine nach Anspruch 1 angegeben.

### Vorteile der Erfindung:

Der Erfindung liegt der Kerngedanke zugrunde, die Vorteile der bekannten, zuvor beschriebenen Systeme zu vereinigen und insbesondere ein regelbares System zu schaffen, bei welchem je nach Bedarf eines der in den Figuren 6 bis 8 beschriebenen Grundprinzipien der angewendeten Narkosesysteme verwendet wird. Dabei wird in aller Regel das sogenannte halbgeschlossene Narkosesystem nach Figur 7 verwendet, wobei Figur 6 und Figur 8 jeweils Grenzfälle darstellen. Es wird demnach stets ein Teil des exspirierten Atemgases des Patienten in den inspiratorischen Zweig zurückgeführt, um eine möglichst geringe Menge von Frischgas aus dem Narkosegerät zu verwenden, wobei die benötigte Menge an Frischgas durch die Applikation und den Bedarf am Patienten bestimmt wird. Hierfür ist es erfindungsgemäß erforderlich, daß das inspiratorische Gas mittels eines angesteuerten Inspirationsventils geregelt und gleichermaßen der Umfang des zirkulierenden Atemweggases über ein entsprechend angesteuertes Auslaßventil ebenfalls geregelt wird. Dem erfindungsgemäßen Anästhesiegerät mit den Baueinheiten Narkosegerät, Narkosesystem sowie Beatmungssystem ist deshalb eine Steuerung oder Regelung zugeordnet, die die verschiedensten Funktionen der einzelnen Bauelemente und insbesondere das Inspirationsventil sowie das Überschußventil oder Auslaßventil steuert oder regelt und damit die optimalen Zustände herbeiführt. Dabei ist es für die Erfindung wesentlich, daß ein Wechseldruck-Gasspeicher vorgesehen ist, der mit hohem Druckniveau die inspiratorische Phase beeinflußt. Hierdurch kann die Regelung des inspiratorischen Gases über das Inspirationsventil sehr feinfühlig und genau durchgeführt werden, wobei Frischgas und/oder wiederverwendbares exspiratorisches Gas aus dem Wechseldruck/Gasspeicher verwendet werden kann.

Für die Erfindung ist es dabei wichtig, daß das exspiratorische und wiederzuverwendete Gas einfach in den Wechseldruck-Gasspeicher während der Exspirationsphase gelangen kann. Dies wird zum einen durch die Einstellung des ansteuerbaren Auslaßventils und zum anderen durch den Unterdruck im Wechseldruck-Gasspeicher selbst bestimmt.

"Wechseldruck" im Sinne der Erfindung bedeutet, daß der Druck im Gasspeicher für die Aufnahme des exspirierten Gases und/oder des Frischgases auf Null oder geringfügig unter Null abgesenkt werden kann und für die Durchführung der Beatmung auf einen definierten Arbeitsdruck vor dem Einsetzen der Inspirationsphase komprimierbar ist.

Neben der genauen Dosierung des inspiratorischen Gasflusses und damit einhergehend auch des inspiratorischen Gasvolumens ergeben sich bei den herkömmlichen Narkosemaschinen im halbgeschlossenen Narkosesystem immer dann erhebliche Probleme, wenn der Patient bei neueren Narkose- und Therapieverfahren längere Zeit spontan aus der Narkosemaschine atmen muß. Die technischen Schwierigkeiten bei dieser Anwendungsform bestehen darin, daß die Narkosemaschine den Gasfluß zum Patienten an das Spontanatemverhalten des Patienten anpassen muß. In besonders schwierigen Situationen ist es darüber hinaus notwendig, dem Patienten nicht nur die notwendige Gasmenge für die Spontanatmung zu liefern, sondern auch noch -bei sehr schwachen Patienten- eine zusätzliche Atemhilfe oder Spontanatmungsunterstützung zu gewähren. Beim erfindungsgemäßen System gelingt dies, indem durch eine Messung des vom Patienten bei der Spontanatmung erzeugten Unterdrucks und/oder Gasflusses die Gaslieferung über das steuer- und regelbare Inspirationsventil erfolgt. Die Meßeinrichtung für den durch den spontan atmenden Patienten erzeugten Unterdruck und/oder Gasfluß wird als Triggereinrichtung bezeichnet.

Weitere Einzelheiten und Vorteile der Erfindung sind in der nachfolgenden Beschreibung von Ausführungsbeispielen erläutert, die anhand der Figurendarstellungen näher beschrieben sind. Es zeigen
- Figur 1a, 1b: ein Standardsystem für ein halboffenes, halbgeschlossenes und geschlossenes Narkosesystem;
- Figur 2a, 2b: ein Einfachsystem für ein halbgeschlossenes Narkosesystem;
- Figur 3a, 3b: ein Narkoseatemsystem bei bestehendem Kreissystemtypen mit integriertem, austauschbaren Inspirationsventil;
- Figur 4a, 4b: eine Ventilkombination für den Anschluß von Narkoseatemsystemen bei bestehenden Kreissystemtypen und
- Figur 5a, 5b: ein System nach der Variation der Figuren 1 bis 4 mit zuschaltbarer Handbeatmung,
wobei die Figur a jeweils den inspiratorischen und die Figur b den exspiratorischen Beatmungsvorgang zeigt.

### Beschreibung von Ausführungsbeispielen der Erfindung:

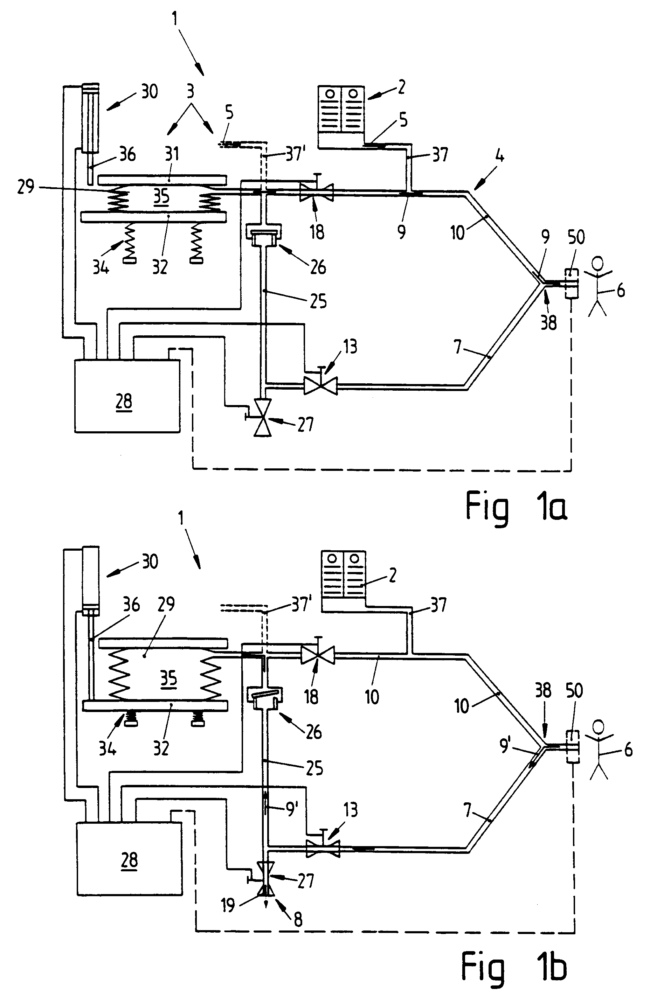

In den Figuren 1 bis 5 sind gleiche Teile mit gleichen Bezugszeichen bezeichnet, sofern sie bei der Beschreibung des Standes der Technik nach den Figuren 6 bis 13 für gleiche Teile bereits verwendet worden sind.

Generell erfolgt die Problemlösung bei allen Ausführungsbeispielen nach Figur 1 bis 5 dadurch, daß grundsätzlich zwischen inspiratorischem Zweig 10 und exspiratorischem Zweig 7 eine Verbindungsleitung 25 ggf. mit Rückschlagventil 26 vorhanden ist, die eine Rückführung des exspiratorischem Atemgases 9' in den inspiratorischen Zweig 10 erlaubt. Für die inspiratorische Phase ist ein ansteuerbares Inspirationsventil 18 sowie ein ansteuerbares Auslaßventil 27 vorhanden, die mit einer Steuer- oder Regeleinheit 28 verbunden sind. Zusätzlich ist ein als Wechseldruck-Gasspeicher ausgebildeter Druckerzeuger 29 als Beatmungsteil 3 vorgesehen, der über eine separate oder integrierte pneumatische oder elektrische Kolbenzylindereinheit 30 bewegbar ist. Mittels der Kolbenzylindereinheit 30 kann im Wechseldruck-Gasspeicher 29 eine Absenkung des Druckes bewirkt werden, um eine problemlose Aufnahme des Exspirationsgases während der Exspirationsphase zu ermöglichen.

Der Wechseldruck-Gasspeicher 29 kann als Druckkonstanthalteeinrichtung jeglicher Bauart ausgebildet sein. In den Ausführungsbeispielen nach Figur 1 bis 5 besteht dieser Gasspeicher aus einer festen Platte 31, sowie einer beweglichen Platte 32 mit einem dazwischenliegenden Federbalg 33. Ein unterhalb der beweglichen Platte 32 angeordnetes Federsystem 34 dient dem selbständigen Antrieb des Gasspeichers. Selbstverständlich kann auch eine zwangsangetriebene Kolbenzylindereinheit zur Herstellung des Gasraumes 35 innerhalb des Gasspeichers 29 verwendet werden.

Die Kolbenzylindereinheit 30 weist eine pneumatisch oder elektrisch angetriebene Kolbenstange 36 auf, die die bewegliche Platte 32 während der exspiratorischen Phase derart mit Kraft beaufschlagt, daß das Gasvolumen 35 vergrößert wird. Dabei erfolgt die Steuerung der Kolbenstange 36 wiederum über entsprechende Zuleitungen zur Steuereinheit 28.

Die in der Figur 1a dargestellte Inspirationsphase arbeitet wie folgt:

Kurz vor Beginn der eigentlichen Inspirationsphase wird die hier beispielhaft gezeigte Kolbenstange 36 in die Ausgangsposition durch die Steuereinheit 28 zurückgefahren, wodurch die bewegliche Platte 32 des Wechseldruck-Gasspeichers 29 freigegeben wird. Die nun wirksam werdende Federkräfte des Federsystems 34 komprimieren den Gasspeicher und erzeugen den für die Inspiration erforderlichen Arbeitsdruck. Dieser Arbeitsdruck liegt in der Größenordnung des zwei- bis dreifachen des üblichen Beatmungsdruckes, d. h. z. B. bei ca. 60 mbar. Durch die Steuerung der Steuereinheit 28 wird für die eigentliche Inspiration des Patienten 6 das Exspirationsventil 13 über eine entsprechende Steuerleitung geschlossen und gleichzeitig das Inspirationsventil so geöffnet, daß zwischen dem Gasspeicher 29 und dem Patienten 6 der gewünschte inspiratorische Gasfluß zustandekommt. Dabei stellt die Lunge des Patienten ein elastischen Gasspeicher dar, in welchen das Atemgas einströmt. Durch dieses in die Lunge einströmende Atemgas erhöht sich der Lungendruck in Abhängigkeit der Gewebeelastizität und der einströmenden Gasmenge. Zur Aufrechterhaltung des inspiratorischen Gasflußes muß daher das Inspirationsventil 18 durch die Steuer- und Regeleinheit 28 entsprechend nachgeregelt werden.

Nach der Applikation des erforderlichen Atemhubvolumens in Abhängigkeit des Patienten wird das Inspirationsventil 18 geschlossen und die Lunge des Patienten für kurze Zeit im geblähten Zustand belassen. Dies wird als sogenannte inspiratorische Pause bezeichnet.

Während der Inspirationsphase kann das aus dem Narkosegerät 2 mit Hochdruckgasversorgung entnommene Frischgas über eine separate Leitung 37 dem inspiratorischen Zweig 10 gesondert zugeführt werden. Dabei kann eine kontinuierliche oder diskontinuierliche Frischgaszuführung 5 über die Leitung 37 erfolgen.

Die Zufuhrleitung 37 für das Frischgas kann gemaß der Darstellung in Figur 1a, 1b zwischen dem Inspirationsventil 18 und dem Patienten-Anschluß-T-Stück 38 liegen. In diesem Fall wird die Frischgaszuführung von der Einstellung des regelbaren Inspirationsventils 18 während der Inspirationsphase nicht berührt. Gleiches gilt für die in Figur 1b dargestellte Exspirationsphase, in welcher die Frischgaszuführung zweckmäßigerweise diskontinuierlich betrieben wird. Auch dieser Betrieb wird durch die Steuer- und Regeleinheit 28 geregelt.

Gemäß der Darstellung nach Figur 1a, 1b kann die Frischgaszuführung 37' für das Frischgas 5 auch zwischen dem Wechseldruck-Gasspeicher 29 und dem Inspirationsventil 18 angeordnet sein, wie dies gestrichelt dargestellt ist. In diesem Fall wird der Gasfluß der Frischgaszuführung durch die Regelung des Inspirationsventils 18 erfaßt, so daß auch Frischgas in den Gasspeicher 29 und damit in das Beatmungsteil 3 gelangen kann.

Die in der Figur 1b dargestellte Exspirationsphase arbeitet wie folgt:

Kurz vor oder gleichzeitig mit der Umschaltung auf die Exspirationsphase, d. h. im Zeitpunkt des Öffnens des Exspirationsventils 13 wird durch die Steuerung bzw. Regelung der Einheit 28 die Kolbenstange 36 ausgefahren und dadurch entgegen den Federkräften des Federsystems 34 die bewegliche Platte 32 nach unten gedrückt. Durch die Volumenvergrößerung des Gasraumes 35 im Gasspeicher 29 sinkt der Druck in diesem Gasspeicher auf Null oder unter Umständen auch auf einen geringfügig unterhalb Null liegenden Unterdruck ab. Nach dem Öffnen des Exspirationsventils 13 strömt aufgrund des positiven Lungendrucks des Patienten 6 das Exspirationsgas über den exspiratorischen Zweig 7 und die Verbindungsleitung 25 über das in diesem Fall geöffnete Rückschlagventil 26 in den Gasspeicher 29. Während dieser Phase erfolgt auch eine Regelung des Auslaßventils 27. Je nach Öffnungsgrad des Auslaßventils oder Überschußventils 27 wird das Exspirationsgas 9' in den Gasspeicher 29 oder aus dem System (Pfeil 19) geleitet. Die nicht benötigte Exspirationsgasmenge kann demzufolge über das Überschußventil in die Narkosegasabsaugung 8 (Pfeil 19) abgeleitet werden. Dies gilt insbesondere in Abhängigkeit des ansteigenden Drucks im Speichersystem.

Bei dem erfindungsgemäßen Gerätesystem liegt demnach der Hauptvorteil in der genauen Regelung des inspiratorischen Gasflusses. Dabei wird vorzugsweise das halbgeschlossene Narkosesystem mit einem Wechseldruck-Gasspeicher 29 verwendet, wobei das exspiratorische Gas dem Gasspeicher teilweise oder vollständig (geschlossenes Narkosesystem) zugeführt wird und nur die nicht benötigte Ausatemmenge in die Narkosegasabsaugung abgeführt wird. Die Regelung erfolgt dabei über den Gasdruck im Gasspeicher 29 und der Regelung des Auslaßventils 27, welches dem Exspirationsventil 13 nachgeschaltet ist. Durch die erfindungsgemäße Kolbenzylindereinheit 30 läßt sich der Zufluß des exspiratorischen Gases in den Gasspeicher 29 problemlos einstellen, wobei auch eine andere angetriebene Gas-Zylindereinrichtung 29 verwendbar ist.

Selbstverständlich kann auch das Exspirationsventil 13 als sogenanntes Peep-Ventil ausgebildet sein.

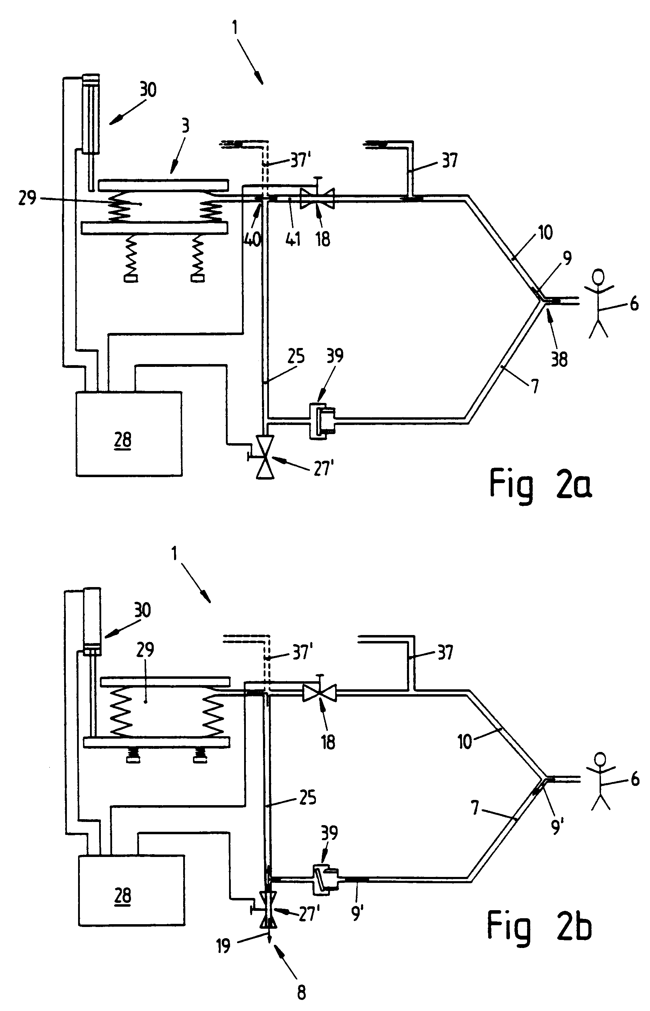

Beim Ausführungsbeispiel der Erfindung nach Figur 2a, 2b kommt ein vereinfachtes System für das halbgeschlossene Narkosesystem zur Anwendung. Dabei sind gleiche Teile mit gleichen Bezugszeichen wie in den Figuren 1a, 1b bezeichnet.

Beim vereinfachten Ausführungsbeispiel nach Figur 2a, 2b wird auf das Rückschlagventil 26 in der Leitung 25 verzichtet, da diese Verbindungsleitung 25 zwischem dem exspiratorischen Zweig 7 und dem inspiratorischen Zweig 10 während der inspiratorischen Phase gemäß Figur 2a auch anderweitig verschlossen werden kann. Hierfür wird das in Figur 1a vorhandene Exspirationsventil 13 durch ein einfaches Einwegventil 39 ersetzt, während das Auslaßventil 27' als Kombiventil, d. h. als kombiniertes Überschuß-, Exspirations- und/oder Peep-Ventil ausgebildet ist. Hierdurch kann während der inspiratorischen Phase gemäß Figur 2a der exspiratorische Zweig 7 und die Umgebung bzw. die Narkoseabgasabsaugung verschlossen bleiben.

Während der exspiratorischen Phase gemäß Figur 2b öffnet sich das Einwegventil 39 so daß das exspiratorische Gas 9' je nach Einstellung des Kombiventils 27' ebenfalls über die Verbindungsleitung 25 in den Gasspeicher 29 oder über das Kombiventil 27' in die Narkosegasabsaugung 8 (Pfeil 19) gelangen kann. Die Funktionsweise dieser Gasführung erfolgt auf gleichem Wege wie zu Figur 1a, 1b beschrieben.

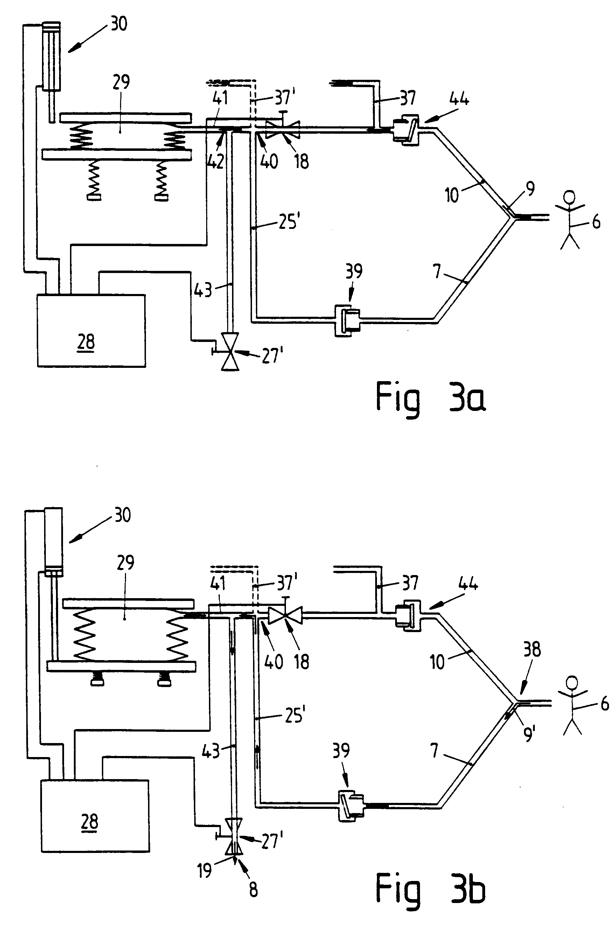

Beim weiterhin vorhandenen Ausführungsbeispiel nach Figur 3a, 3b ist ein Narkoseatemsystem nach der Bauart nach Figur 2a, 2b vorgesehen, bei welchem bestehende Kreissystemtypen mit integriertem, austauschbaren Inspirationsventil 18 verwendet werden. Dabei sind wiederum gleiche Teile mit gleichen Bezugszeichen bezeichnet.

Gegenüber dem Ausführungsbeispiel nach Figur 2a, 2b ist bei dem Ausführungsbeispiel nach Figur 3a, 3b eine erste Gasrückführleitung 25' im bestehenden Kreissystem vorgesehen, die eine kreisförmige Leitung bildet, wie dies in Figur 8 für das geschlossene Narkosesystem dargestellt ist. Das exspiratorische Gas 9' wird demnach grundsätzlich über das Einwegventil 39 in den inspiratorischen Zweig 10 zurückgeführt und gelangt über ein T-Stück 40 in die inspiratorische Leitung 41 zwischem Gasspeicher 29 und dem regelbaren Inspirationsventil 18, wie dies auch im Ausführungsbeispiel nach Figur 1 und 2 der Fall ist. Im Gegensatz zu den beiden genannten Ausführungsbeispielen ist jedoch das Kombiventil 27' nicht im exspiratorischen Zweig 7 sondern im inspiratorischen Zweig 10 über ein weiteres T-Stück 42 an die inspiratorische Leitung 41 zwischen Gasspeicher 29 und Inspirationsventil 18 angeschlossen, so daß sich eine zusätzliche Gasleitung 43 für den exspiratorischen Zweig ergibt.

Während der inspiratorischen Phase wird demnach sowohl das Rückschlagventil 39 als auch das Kombiventil 27' geschlossen, so daß das inspiratorische Gas über den inspiratorischen Zweig 41, 10 aus dem Gasspeicher 29 sowie der zusätzlichen Frischgaszuführung 37 bzw. 37' zum Patienten 6 gelangt. Dies entspricht der Gasführung gemäß dem Ausführungsbeispiel nach Figur 2a.

Während der exspiratorischen Phase gemäß der Darstellung nach Figur 3b öffnet sich das Rückschlagventil 39 und das exspiratorische Atemgas gelangt über die Verbindungsleitung 25' zum T-Stück 40 und von dort über die Leitung 41 in den Gasspeicher 29. Je nach der Stellung des regelbaren Kombiventils 27' erfolgt eine Ableitung des exspiratorischen Überschußgases über die zusätzliche Gasleitung 43 in die Narkosegasabsaugung 8 (Pfeil 19). In dieser exspiratorischen Phase kann der Inspirationszweig über ein zusätzliches Einwegventil 44 geschlossen sein. Dieses Einwegventil 44 ist zwischen dem Patienten-Anschluß-T-Stück 38 und dem Inspirationsventil 18 angeordnet. Zwischem dem Einwegventil 44 und dem Inspirationsventil 18 befindet sich die Frischgaszuführung 37 des nicht näher dargestellten Narkosegerätes 2.

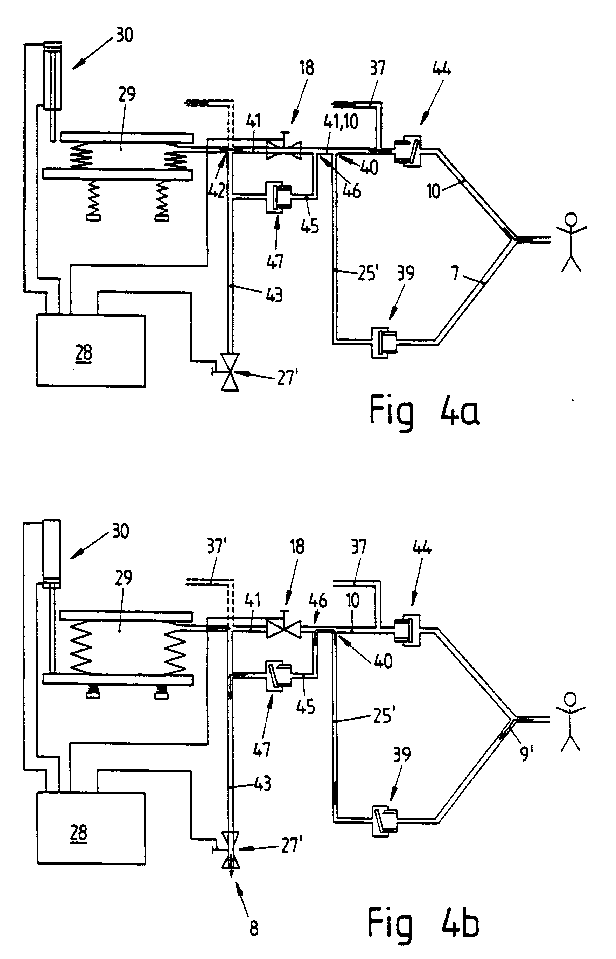

In der Figur 4a, 4b ist ein weiteres Ausführungsbeispiel der Erfindung in Abwandlung des Ausführungsbeispiels nach Figur 3a, 3b dargestellt. Gleiche Teile sind mit gleichen Bezugszeichen gekennzeichnet. Diese Ventilkombination dient für den Anschluß eines Narkoseatemsystems bei bestehenden Kreissystemtypen.

In Weiterbildung bzw. Abwandlung des Systems nach Figur 3a, 3b befindet sich das Inspirationsventil 18 außerhalb des Kreissystems. Gemäß dem Ausführungsbeispiel der Erfindung nach Figur 4a, 4b wird das exspiratorische Gas in einer zu Leitung 41 parallel geschalteten Leitung 45 vom T-Stück 40 im inspiratorischen Zweig 10 über ein weiteres T-Stück 46 der Parallelleitung 45 zugeführt, um das Inspirationsventil 18 zu umgehen. In dieser Parallelleitung 45 befindet sich ein weiteres Einwegventil 47, welches während der inspiratorischen Phase nach Figur 4a geschlossen und während der exspiratorischen Phase nach Figur 4b geöffnet ist. Insofern kann während der inspiratorischen Phase das inspiratorische Gas bei geschlossenem Einwegventil 47 und geschlossenem Kombiventil 27' nur über das ansteuerbare Inspirationsventil 18 zum Patienten gelangen.

Während der Exspirationsphase nach Figur 4b gelangt das exspiratorische Gas 9' über das Einwegventil 39 und die Rückführleitung 25 in den inspiratorischen Zweig 10 und von dort aus über die Verbindungsleitung 45, das Einwegventil 47 in die zusätzliche Gasleitung 43. Je nach Öffnung des Kombiventils 27' erfolgt wiederum eine Zufuhr des Exspirationsgases zum Gasspeicher 29 oder über das Kombiventil 27' zur Narkosegasabsaugung 8. Ggf. kann die Leitung 25' direkt mit der Leitung 45 verbunden werden.

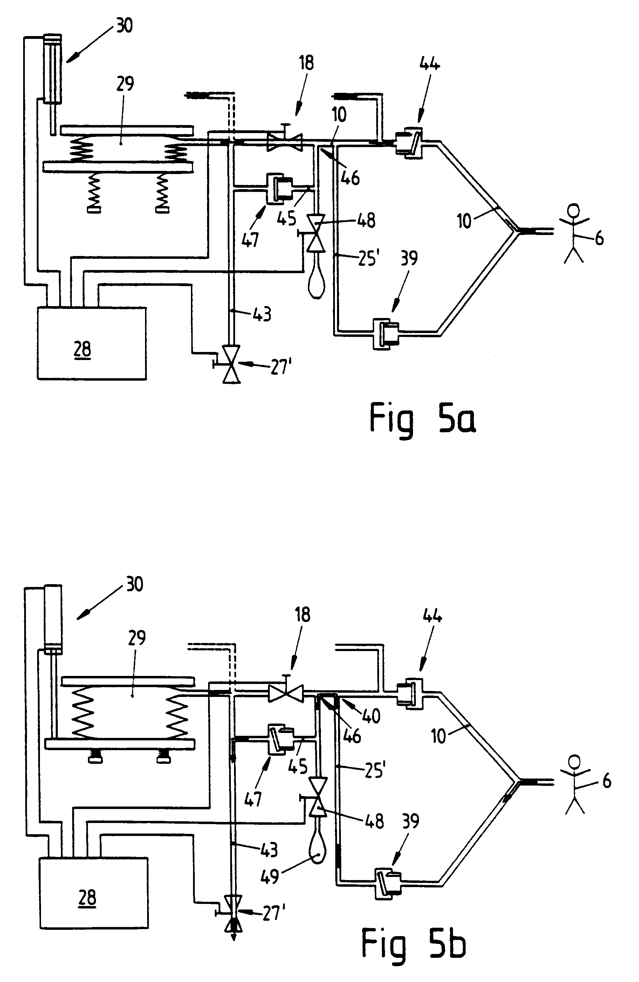

Das Ausführungsbeispiel der Erfindung nach Figur 5a, 5b stellt eine einfache Weiterbildung des Ausführungsbeispiels nach Figur 4a, 4b dar. Gleiche Teile sind mit gleichem Bezugszeichen versehen. Beim Ausführungsbeispiel nach Figur 5a, 5b kann das System nach Figur 4, prinzipell auch das System der Figuren 1 bis 3 mit einer zuschaltbaren Handbeatmung versehen werden. Hierfür ist an die Leitung 45 ein weiteres regelbares Ventil 48 angeschlossen, welches zum inspiratorischen Zweig über das obere T-Stück 46 führt. Hierdurch kann während der inspiratorischen Phase zusätzlich mittels eines Handbalgs 49 eine handgesteuerte Beatmung des Patienten durchgeführt werden, soweit das regelbare Ventil 48 geöffnet ist.

Während der exspiratorischen Phase kann das Ventil 48 offen sein, damit Frischgas und ggf. exspiratorisches Gas in den Handbeatmungsbalg 49 gelangen kann.

In Figur 1a ist zur Erfassung der Spontanatemtätigkeit des Patienten noch eine patientennahe Meßeinrichtung 50 für den durch die Spontanatmung erzeugten Unterdruck und/oder den Gasfluß angedeutet. Diese "Triggereinrichtung" steuert das regelbare Inspirationsventil 18 an und erzeugt eine zusätzliche Atemhilfe oder Spontanatmungsunterstützung.

Die Erfindung ist nicht auf die dargestellten und beschriebenen Ausführungsbeispiele beschränkt. Sie umfaßt auch vielmehr alle fachmännischen Weiterbildungen im Rahmen der Schutzrechtsansprüche. Insbesondere kann der Wechseldruck-Gasspeicher 29 auch als Kolben/Zylindereinheit mit einem eigenen regel- oder steuerbaren Antrieb in beiden Richtungen ausgebildet sein.
- 1: Anästhesiemaschine
- 2: Narkosegerät mit Hochdruckgasversorgung
- 3: Beatmungsteil
- 4: Narkosesystem
- 5: Frischgasfluß
- 6: Patient
- 7: Leitung exsp. Zweig
- 8: Narkoseabsaugung
- 9: Atemgas exsp.
- 10: Zuführleitung insp. Zweig
- 11: Kreislauf
- 12: Pfeil
- 13: Exspirationsventil
- 14: Druckgasspeicher
- 15: feste Platte
- 16: bewegliche Platte
- 17: Druckfedersystem
- 18: ansteuerbares Insp.
- 19: Pfeil
- 20: Kolbenpumpe
- 21: "Bag in the bottle"-Prinzip
- 22: Rückschlagventil
- 23: Beutel
- 24: Behälter
- 25: Verbindungsleitung
- 26: Rückschlagventil
- 27: Auslaßventil
- 28: Regel/Steuereinheit
- 29: Wechseldruck-Gasspeicher/Druckerzeuger
- 30: Kolben-Zylindereinheit
- 31: feste Platte
- 32: bewegliche Platte
- 33: Federbalg
- 34: Federsystem
- 35: Gasraum
- 36: Kolbenstange
- 37: Leitung
- 38: Patienten-Anschluß-T-Stück
- 39: Einweg-Ventil
- 40: T-Stück
- 41: Leitung
- 42: T-Stück
- 43: Gasleitung
- 44: Einweg-Ventil
- 45: Leitung
- 46: T-Stück
- 47: Einweg-Ventil
- 48: regelbares Ventil
- 49: Handbalg
- 50: Meßeinrichtung für Unterdruck/Gasfluß / Triggereinrichtung

## Patentansprüche

1. Anästhesiemaschine bestehend aus einem Narkosegerät (2) zur Lieferung von Frischgas (5), aus einem Beatmungsteil (3) zur Unterstützung oder Durchführung der Beatmung des Patienten sowie einem Narkosesystem (4) zur Durchführung des eigentlichen Gasaustausches zwischen Maschine und Patient, wobei das Narkosesystem (4) einen inspiratorischen (10) und einen exspiratorischen Zweig (7) aufweist, die über einen Patientenanschluß T-Stück (38) miteinander verbunden sind, mit einem im inspiratorischen Zweig (10) angeordneten Druckerzeuger (29) für den Beatmungsteil (3) und einem nachgeschalteten Inspirationsventil (18) und einem im exspiratorischen Zweig (7) angeordneten Exspirationsventil bzw. Auslaßventil (13, 27) und mit einer Verbindungsleitung (25, 25') zwischem dem inspiratorischem und dem exspiratorischen Zweig (7, 10) zur Herstellung eines halbgeschlossenen, eines geschlossenen oder eines halboffenen Narkosesystems, dadurch gekennzeichnet, daß der Druckerzeuger (29) als Wechseldruck-Gasspeicher (29) ausgebildet ist, welchem eine steuerbare Antriebseinrichtung (30) wenigstens zur Vergrößerung des Hubvolumens des Gasspeichers (29) zugeordnet ist, daß das exspiratorische Atemgas (9') des Patienten (6) über den exspiratorischen Zweig (7) und über die Verbindungsleitung (25, 25') in einstellbarer Menge dem Gasspeicher (29) zuführbar ist, wobei ein steuer- oder regelbares Überschußventil (27) im exspiratorischen Zweig (7) die abzuleitende Atemgasmenge bestimmt und daß der unter Druck gesetzte Gasinhalt des Gasspeichers (29) in der inspiratorischen Phase über ein steuer- oder regelbares Inspirationsventil (18) entnehmbar ist.

2. Maschine nach Anspruch 1, dadurch gekennzeichnet, daß das Atemgas für die Durchführung der Inspiration unter hohem Druck im Gasspeicher (29) bereitgehalten und über das Inspirationsventil (18) geregelt wird und daß der Druck im Gasspeicher (29) während der Exspirationsphase soweit abgesenkt ist, daß das vom Patienten über die Verbindungsleitung (25) zurückströmende Exspirationsgas ganz oder teilweise im Gasspeicher (29) gesammelt wird.

3. Maschine nach Anspruch 1, dadurch gekennzeichnet, daß bei teilweiser Wiederverwendung des Exspirationsgases im Gasspeicher (29) das überschüssige Exspirationsgas über das Überströmventil (27) abgeleitet wird.

4. Maschine nach Anspruch 1, 2 oder 3, dadurch gekennzeichnet, daß das Exspirationsventil (13) als Peep-Ventil einen dauernden positiven Atemwegsdruck erzeugt.

5. Maschine nach einem der vorhergehenden Ansprüche, dadurch gekennzeichnet, daß die Frischgaszuführung zum Patienten über eine Zuführleitung (37) im inspiratorischen Zweig (10) erfolgt, die vor oder nach dem Inspirationsventil (18) angeordnet ist.

6. Maschine nach Anspruch 5, dadurch gekennzeichnet, daß die Frischgaszuführung zum Gasspeicher (29) und/oder zum Patienten (6) über eine Frischgaszuführleitung (37, 37') kontinuierlich oder diskontinuierlich erfolgt.

7. Maschine nach einem der vorhergehenden Ansprüche, dadurch gekennzeichnet, daß der Gasspeicher (29) eine feststehende (31) sowie eine bewegliche Platte (32) aufweist, wobei die bewegliche Platte (32) über ein Federsystem (34) im Sinne einer Verkleinerung des Hubvolumens antreibbar ist.

8. Maschine nach einem der vorhergehenden Ansprüche, dadurch gekennzeichnet, daß eine Druckentlastung und/oder der Antrieb des Gasspeichers (29) durch eine pneumatische oder elektrische Stelleinrichtung und insbesondere eine pneumatische oder elektrische Kolben/Zylindereinheit (30) erfolgt.

9. Maschine nach einem der vorhergehenden Ansprüche, dadurch gekennzeichnet, daß die Verbindungsleitung (25) ein Rückschlagventil (26) aufweist, welches während der inspiratorischen Phase geschlossen ist.

10. Maschine nach einem der vorhergehenden Ansprüche, dadurch gekennzeichnet, daß im exspiratorischen Zweig ein Exspirationsventil (13) und ein Überschußventil (27) als regelbare Ventile angeordnet sind, wobei die Verbindungsleitung (25) zwischen den Ventilen (13, 27) abzweigt.

11. Maschine nach einem der vorhergehenden Ansprüche, dadurch gekennzeichnet, daß im exspiratorischen Zweig ein Rückschlagventil (39) vorgesehen ist, welches während der inspiratorischen Phase den exspiratorischen Zweig (7) gegenüber dem inspiratorischen Zweig (10) verschließt und daß das Überschußventil als kombiniertes Überschuß-, Exspirations- und/oder Peep-Ventil (27') ausgebildet ist.

12. Maschine nach einem der vorhergehenden Ansprüche, dadurch gekennzeichnet, daß der exspiratorische Zweig (7) über eine Verbindungsleitung (25') zum inspiratorischen Zweig (10) zur Atemgasrückführung zum Gasspeicher (29) führt und daß am inspiratorischen Zweig (10) ein kombiniertes Überschuß, Exspirations- und/oder Peep-Ventil (27') angeschlossen ist.

13. Maschine nach Anspruch 12, dadurch gekennzeichnet, daß die Rückführleitung (25') vom exspiratorischen (7) zum inspiratorischen Zweig (10) zwischen Patient und Rückschlagventil (18) im inspiratorischen Zweig (10) mündet und daß eine Bypassleitung (45) mit Rückschlagventil (47) das regelbare Inspirationsventil (18) überbrückt, wobei das Rückschlagventil (47) in der Inspirationsphase geschlossen ist.

14. Maschine nach einem der vorhergehenden Ansprüche, dadurch gekennzeichnet, daß eine zuschaltbare Handbeatmungseinrichtung (49) mit Schaltventil (48) dem inspiratorischen Zweig (10) zugeordnet ist.

15. Maschine nach einem der vorhergehenden Ansprüche, dadurch gekennzeichnet, daß der Wechseldruck-Gasspeicher (29) in Verbindung mit dem regelbaren Inspirationsventil (18) und einer zusätzlichen Triggereinrichtung (50) den Patienten-Gasbedarf eines spontanatmenden Patienten liefern kann.

16. Maschine nach Anspruch 15, dadurch gekennzeichnet, daß die Triggereinrichtung (50) den Patienten bei einer Spontanatmung eine inspiratorische Atemhilfe gewähren kann.

## Claims

1. Anaesthetist's equipment comprising a narcotization device (2) for supplying make-up gas (5), a respirator (3) for supporting or performing the respiration of the patient and a narcotization system (4) for performing the actual gas exchange between the equipment and the patient, in which the narcotization system (4) comprises an inspiratory (10) and an expiratory arm (7) which are connected together by a patient T-shaped connection tube (38), with a pressure generator (29) arranged in the inspiratory arm (10) for the respirator (3) and a downstream inspiration valve (18) and an expiration valve or outlet valve (13, 27) arranged in the expiratory arm (7) and with a connecting line (25, 25') between the inspiratory and the expiratory arm (7, 10) for producing a half-closed, closed or half-open narcotization system, characterised in that the pressure generator (29) is designed as an alternating pressure gas storage unit (29) to which a controllable drive device (30) is assigned at least for increasing the stroke volume of the gas storage unit (29), the expiratory gas (9') of the patient (6) can be guided via the expiratory arm (7) and via the connecting line (25, 25') in an adjustable amount to the gas storage unit (29), whereby a controllable or regulatable overflow valve (27) in the expiratory arm (7) determines the amount of respiratory gas to be drawn off and the gas content of the gas storage unit (29) put under pressure in the inspiration phase can be removed via a controllable or regulatable inspiration valve (18).

2. Equipment according to claim 1, characterised in that the respiratory gas for performing the inspiration at high pressure in the gas storage unit (29) is kept ready and is controlled by the inspiration valve (18) and the pressure in the gas storage unit (29) is reduced during the expiration phase so much that the expiratory gas flowing back from the patient via the connecting line (25) is collected completely or partly in the gas storage unit (29).

3. Equipment according to claim 1, characterised in that on partly recycling the expiration gas in the gas storage unit (29) the excess expiration gas is removed via the overflow valve (27).

4. Equipment according to claim 1, 2 or 3, characterised in that the expiration valve (13) as a peep valve generates a continually positive respiratory path pressure.

5. Equipment according to one of the preceding claims, characterised in that the make-up gas is supplied to the patient via a supply line (37) in the inspiratory arm (10) which is arranged in front or after the inspiration valve (18).

6. Equipment according to claim 5, characterised in that the make-up gas is supplied continually or discontinually to the gas storage unit (29) and/or to the patient (6) via a make-up gas supply line (37, 37').

7. Equipment according to one of the preceding claims characterised in that the gas storage unit (29) comprises a fixed (31) and a moving plate (32), whereby the moving plate (32) can be driven by a spring system (34) to reduce the stroke volume.

8. Equipment according to one of the preceding claims, characterised in that pressure relief and/or the drive of the gas storage unit (29) is performed by a pneumatic or electric control device and in particular a pneumatic or electric piston/cylinder unit (30).

9. Equipment according to one of the preceding claims, characterised in that the connecting line (25) comprises a check valve (26) which is closed during the inspiration phase.

10. Equipment according to one of the preceding claims, characterised in that in the expiratory arm an expiration valve (13) and an overflow valve (27) are arranged as controllable valves whereby the connecting line (25) branches between the valves (13, 27).

11. Equipment according to one of the preceding claims, characterised in that in the expiratory arm a check valve (39) is provided which during the inspiration phase closes the expiratory arm (7) from the inspiratory arm (10) and the overflow valve is designed as a combined overflow, expiration and/or peep valve (27').

12. Equipment according to one of the preceding claims, characterised in that the expiratory arm (7) leads via a connecting line (25') to an inspiratory arm (10) to the respiration gas return to the gas storage unit (29) and to the inspiratory arm (10) a combined overflow, expiration and/or peep valve (27') is connected.

13. Equipment according to claim 12, characterised in that the return line (25') from the expiratory (7) to the inspiratory arm (10) between the patient and check valve (18) exits in the inspiratory arm (10) and a bypass line (45) with a check valve (47) bridges the controllable inspiration valve (18) whereby the check valve (47) is closed in the inspiration phase.

14. Equipment according to one of the preceding claims, characterised in that a connectable hand respiration device (49) with an on-off valve (48) is assigned to the inspiratory arm (10).

15. Equipment according to one of the preceding claims, characterised in that the alternating pressure gas storage unit (29) in connection with the controllable inspiration valve (18) and an additional trigger device (50) can supply the gas requirement of a spontaneously breathing patient.

16. Equipment according to claim 15, characterised in that the trigger device (50) can provide the patient with spontaneous respiration with an inspiration respiratory aid.

## Revendications

1. Appareil d'anesthésie consistant en un appareil narcotique (2) servant à délivrer un gaz frais (5), en une partie respiratoire (3) servant à soutenir ou à exécuter la respiration du patient et en un système narcotique (4) servant à réaliser l'échange proprement dit des gaz entre la machine et le patient, le système narcotique (4) présentant une branche d'inspiration (10) et une branche d'expiration (7), qui sont reliées l'une à l'autre au moyen d'une pièce en T (38) de raccordement au patient, avec un générateur de pression (29) disposé dans la branche (10) d'inspiration pour la partie respiratoire (3) et une soupape d'inspiration (18), montée en aval, et une soupape d'expiration et soupape d'échappement (13, 27) disposée dans la branche d'expiration (7) et avec une conduite de liaison (25, 25') entre la branche d'inspiration et la branche d'expiration (7, 10) servant à réaliser un système narcotique à moitié fermé, un système narcotique fermé ou un système narcotique à demi ouvert,
caractérisé en ce que
le générateur de pression (29) est constitué sous la forme d'un accumulateur de gaz à pression alternée, auquel est associé un dispositif d'entraînement commandable (30) pour augmenter au moins le volume engendré de l'accumulateur de gaz (29), en ce que le gaz d'expiration (9') du patient (6) peut être amené via la branche d'expiration (7) et via la conduite de liaison (25, 25') en une quantité réglable, à l'accumulateur de gaz (29), une soupape de trop-plein (27), que l'on peut commander ou régler, déterminant dans la branche d'expiration (7) la quantité de gaz respiratoire à évacuer et en ce que le contenu, mis sous pression, de l'accumulateur de gaz (29) peut être extrait dans la phase d'inspiration, via une soupape d'inspiration (18) que l'on peut commander ou régler.

2. Appareil selon la revendication 1,
caractérisé en ce que
le gaz de respiration est maintenu prêt pour la réalisation de l'inspiration sous haute pression dans l'accumulateur de gaz (29) et est réglé au moyen de la soupape d'inspiration (18) et, en ce que l'on abaisse la pression dans l'accumulateur de pression (29) pendant la phase d'expiration jusqu'à ce que le gaz d'expiration refoulé via la conduite de liaison (25) par le patient soit complètement ou partiellement rassemblé dans l'accumulateur de gaz (29).

3. Appareil selon la revendication 1,
caractérisé en ce que
lors de la réutilisation partielle du gaz d'expiration dans l'accumulateur de gaz (29), on prélève le gaz d'expiration en excès via la soupape de trop plein (27).

4. Appareil selon la revendication 1, 2 ou 3,
caractérisé en ce que
la soupape d'expiration (13) produit comme soupape de regard une pression de respiration positive permanente.

5. Appareil selon l'une quelconque des revendications précédentes,
caractérisé en ce que
l'amenée du gaz frais au patient a lieu au moyen d'une conduite d'amenée (37) dans la branche d'inspiration (10), qui est disposée avant ou après la soupape d'inspiration (18).

6. Appareil selon la revendication 5,
caractérisé en ce que
l'amenée du gaz frais à l'accumulateur de gaz (29) et/ou au patient (6) a lieu au moyen d'une conduite d'amenée de gaz frais (37, 37'), de façon continue, ou discontinue.

7. Appareil selon l'une quelconque des revendications précédentes,
caractérisé en ce que
l'accumulateur de gaz (29) présente une plaque fixe (31) et une plaque mobile (32), la plaque mobile (32) pouvant être entraînée au moyen d'un système élastique (34) dans le sens d'une diminution du volume engendre.

8. Appareil selon l'une quelconque des revendications précédentes,
caractérisé en ce que
une décharge de pression et/ou l'entraînement de l'accumulateur de gaz (29) a lieu au moyen d'un dispositif de réglage pneumatique ou électrique et en particulier d'un vérin pneumatique ou électrique (30).

9. Appareil selon l'une quelconque des revendications précédentes,
caractérisé en ce que
la conduite de liaison (25) présente un clapet anti-retour (26) qui est fermé pendant la phase d'inspiration.

10. Appareil selon l'une quelconque des revendications précédentes,
caractérisé en ce que
dans la branche d'expiration sont disposés une branche d'expiration (13) et une soupape de trop-plein (27) en tant que soupapes réglables, la conduite de liaison (25) partant en dérivation entre les soupapes (13, 27).

11. Appareil selon l'une quelconque des revendications précédentes,
caractérisé en ce que
dans la branche d'expiration, on prévoit un clapet anti-retour (39), qui pendant la phase d'inspiration ferme la branche d'expiration (7) par rapport à la branche d'inspiration (10) et en ce que la soupape de trop-plein est constituée comme une soupape combinée de trop-plein d'expiration et/ou de regard (27').

12. Appareil selon l'une quelconque des revendications précédentes,
caractérisé en ce que
la branche d'expiration(7) mène par l'intermédiaire d'une conduite de liaison (25') à la branche d'inspiration (11) pour ramener le gaz de la respiration à l'accumulateur de gaz (29) et en ce que l'on raccorde à la branche d'inspiration (10) une soupape combinée de trop-plein d'expiration et/ou de regard (27').

13. Appareil selon la revendication 12,
caractérisé en ce que
la conduite de retour (25') allant de la branche d'expiration (7) à la branche d'inspiration (10) entre le patient et le clapet anti-retour (18) débouche dans la branche d'inspiration (10) et en ce qu'une conduite de dérivation (45) avec le clapet anti-retour (47) enjambe la soupape d'inspiration réglable (18), le clapet anti-retour (47) étant fermé dans la phase d'inspiration.

14. Appareil selon l'une quelconque des revendications précédentes,
caractérisé en ce qu'
un dispositif de respiration manuel (49) avec soupape de branchement (48) est associé à la branche d'inspiration (10).

15. Appareil selon l'une quelconque des revendications précédentes,
caractérisé en ce que
l'accumulateur de gaz à pression alternée (29) peut, en liaison avec la soupape d'inspiration réglable (18) et un dispositif additionnel de déclenchement (50), délivrer la quantité de gaz dont a besoin un patient respirant spontanément.

16. Appareil selon la revendication 15,
caractérisé en ce que
le dispositif de déclenchement (50) peut assurer au patient, dans le cas d'une respiration spontanée, une aide respiratoire d'inspiration.
